# EUROPEAN PATENT APPLICATION

(11) **EP 4 303 559 A1**
(43) Date of publication of application: **10.01.2024**
(21) Application number: 23183998.6
(22) Date of filing: 06.07.2023
(51) Int. Cl.: G01N 1/22

(54) **AEROSOL DETECTION SYSTEM AND METHOD**

(30) Priority: 07.07.2022 US 202263367893 P; 29.08.2022 US 202263402030 P; 23.09.2022 US 202217952104
(71) Applicant: Hamilton Sundstrand Corporation, Charlotte, NC 28217-4578 (US)
(72) Inventor: GARDNER, Ben D., Colton, CA (US)
(74) Representative: Dehns

(57) **Abstract**

In accordance with at least one aspect of this disclosure, a system includes, a collection arm (110) configured to collect an aerosol sample from a cloud and a detection module (108) operatively connected to the collection arm configured to analyze a composition of the sample from the cloud. In certain embodiments, one or more of the collection arm and/or the detection module can be configured to couple to a mobile platform (104), including for example, a land vehicle, aircraft, or watercraft. In certain embodiments one or more of the collection arm and/or the detection module can be configured to couple to a stationary structure, for example a building.

## Description

### TECHNICAL FIELD

The present disclosure relates to aerosol detection systems and more particularly to chemical and/or biological aerosol detection systems.

### BACKGROUND

There is a need within the national security environment to detect aerosolized chemical threats over a battlespace, or even urban areas. The need includes rapid identification of chemical cloud constituents as well as mapping of cloud dimensions and direction of travel. As a practical matter there is also a strong desire to have the detection system be a "standoff" type wherein the detection system does not come in contact with the cloud and thus become contaminated. Keeping the system free of contamination from chemical weapons agents (CWAs) or other dangerous chemicals eliminates potential post-mission hazards to personnel as they recover the system for reuse.

To date, while several chemical detection systems have been flown on UAVs and have demonstrated the detection of aerosols, there are no suitable systems that can provide both true standoff capability and strong chemical identification capability. The most capable systems (e.g., mobility spectrometry and mass spectrometry) work but require contact with the sample and result in a contaminated system. Optically based systems can be operated from a safe distance but do not have strong detection limits and have limited detection specificity, and to date, these systems only work well analyzing liquid or solid chemicals on stationary surfaces. Chemicals found in aerosol clouds can be highly dispersed and the light detected from them is also highly dispersed, so the limits of detection are greatly diminished.

There remains a need in the art for improvements to chemical aerosol detection. This disclosure provides a solution for this need.

### SUMMARY

In accordance with at least one aspect of this disclosure, a system includes, a collection arm configured to collect an aerosol sample from a cloud and a detection module operatively connected to the collection arm configured to analyze a composition of the sample from the cloud. In certain embodiments, one or more of the collection arm and/or the detection module can be configured to couple to a mobile platform, including for example, a land vehicle, aircraft, or watercraft. In certain embodiments one or more of the collection arm and/or the detection module can be configured to couple to a stationary structure, for example a building.

In embodiments, the collection arm can define a plenum between an outward projection of the collection arm and the detection module. The collection arm can include an opening configured to receive aerosol particles from the cloud and into the plenum. The collection arm can also include a collection tube fluidly connected to the plenum to collect the sample.

A collection medium can be disposed at an inlet of the collection tube configured to collect aerosol particles from the cloud and condense the aerosol particles onto the collection medium. In embodiments, the collection medium can include a filter medium such as a porous medium.

In certain embodiments, a pump can be disposed in the collection tube downstream of the collection medium configured to draw the aerosol particles through the opening in the collection arm, through the inlet of the collection tube, and into or onto the collection medium. In certain embodiments, collection of the sample can be passive. In embodiments, the mobile platform and detection module can be coupled so as to define a fluid channel therebetween to allow fluid from the plenum to exhaust/vent through an outlet of the collection tube, whether passively or actively as pump exhaust.

In embodiments, the detection module includes an optical detection module. The optical detection module can include a radiation source configured to transmit an interrogation beam through the plenum to interrogate the collection medium. The optical detection module can also include a radiation receiver configured to receive a return signal through the plenum from the collection medium. In embodiments, the optical detection module can include a composition determination module configured to receive data from the radiation receiver to determine a chemical composition of the aerosol particles in the collection medium.

In embodiments, a length of the collection tube can be configured to provide a sufficient standoff distance between the mobile platform and detection module and the cloud such that exposure of the mobile platform and detection module to the cloud is minimized. In embodiments, a shape of the collection arm is configured to enclose (e.g., fully) a path of the interrogation beam and a path of the return signal within the plenum. In embodiments, the collection tube can be L-shaped, having a short end and a long side. The inlet of the collection tube can be disposed in the short end of the collection tube and on an opposite end of the long side of the collection tube than the detection module. In embodiments, the inlet of the collection tube can be disposed on an inner surface of the collection arm, proximate the opening in the collection arm, and the collection medium can be disposed in the inlet of the collection tube.

In certain embodiments, the mobile platform further includes a communications module configured to communicate with a controller to guide the mobile platform to one or more positions in space relative to the cloud. In certain embodiments, the controller can include a remote and/or on-ground controller. In certain embodiments, the communications module can be operatively connected to the optical detection module and further configured to communicate the composition of the cloud to the controller and/or a user. In embodiments, the mobile platform can include a navigation module configured to guide the mobile platform to one or more positions in space relative to the cloud without communication from a remote or on-ground controller. In certain embodiments, the mobile platform can be at least partially autonomous.

In accordance with at least one aspect of this disclosure, a method can include remotely collecting sample material from an aerosol cloud in a collection medium, condensing the aerosolized sample in the collection medium, optically interrogating the sample material in the collection medium, determining a chemical composition of the sample material in the collection medium, and communicating the chemical composition to a user and/or a controller. In embodiments, the sample material can include aerosol particles from the aerosol cloud.

In embodiments, remotely collecting the sample can further include detecting an edge of the aerosol cloud, positioning a mobile platform having an optical detection module and a collection arm operatively connected thereto proximate the aerosol cloud separated by a standoff distance such that only a portion of the collection arm enters the aerosol cloud and no portion of the mobile platform or optical detection module enters the aerosol cloud. Collecting the sample can also include drawing a portion of the aerosol cloud through an inlet of a collection tube of the collection arm, wherein the collection medium is disposed at an inlet of the collection tube.

In embodiments, the method can also include moving the mobile platform away from the aerosol cloud while the optical detection system determines the chemical composition, re-positioning the mobile platform proximate the aerosol cloud and repeating the method or homing the mobile platform to a base, and initiating a decontamination protocol based at least in part of the chemical composition of the sample material.

These and other features of the embodiments of the subject disclosure will become more readily apparent to those skilled in the art from the following detailed description taken in conjunction with the drawings.

### BRIEF DESCRIPTION OF THE DRAWINGS

So that those skilled in the art to which the subject disclosure appertains will readily understand how to make and use the devices and methods of the subject disclosure without undue experimentation, embodiments thereof will be described in detail herein below with reference to certain figures, wherein:
Fig. 1 is a schematic diagram of an embodiment of a mobile aerosol collection and detection system in accordance with this disclosure;
Fig. 2 is an enlarged schematic view of an embodiment of a mobile aerosol collection and device in accordance with this disclosure, e.g., for use in the system shown in Fig. 1;
Figs. 3 and 4 include a schematic animation of the system of Fig. 1 performing an embodiment of a method in accordance with this disclosure;
Fig. 5 is a schematic diagram of an embodiment of a stationary aerosol collection and detection system in accordance with this disclosure; and
Fig. 6 is a schematic environmental depiction of the system of Fig. 6, showing the stationary aerosol collection and detection system in an urban environment.

### DETAILED DESCRIPTION

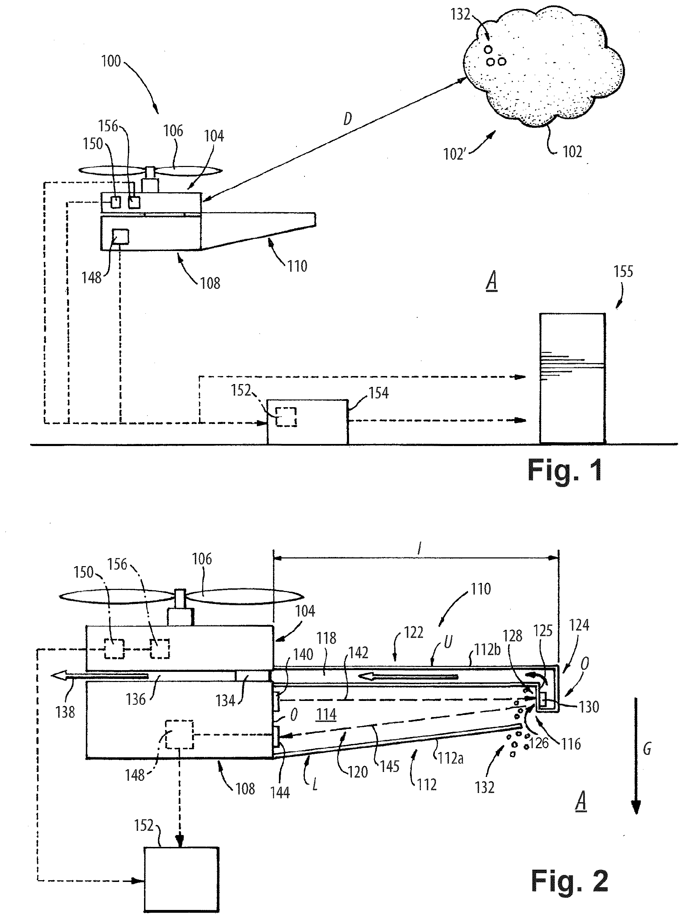

Reference will now be made to the drawings wherein like reference numerals identify similar structural features or aspects of the subject disclosure. For purposes of explanation and illustration, and not limitation, an illustrative view of an embodiment of a system in accordance with the disclosure is shown in Fig. 1 and is designated generally by reference character 100. Other embodiments and/or aspects of this disclosure are shown in Figs. 2-6. Certain embodiments described herein can be used to improve collection and detection of chemical and/or biological threats in the air.

In accordance with at least one aspect of this disclosure, as shown in Figs. 1-4, a system 100 (e.g., for determining a chemical composition of an aerosol cloud 102) includes a vehicle 104. In certain embodiments, e.g., as shown, the vehicle can be an aircraft include one or more air movers 106 operatively connected thereto for moving the vehicle 104. In certain such embodiments, the vehicle can include any suitable aircraft, such as an airplane or a rotorcraft, and may be an unmanned aerial vehicle (UAV). Any suitable vehicle, manned or unmanned, is contemplated herein, including any suitable landcraft, aircraft, or watercraft. In certain embodiments, the moving platform can be a human or robotics. A detection module 108 can be mounted to the vehicle 104 configured to analyze a composition of sample collected from the aerosol cloud 102.

A collection arm 110 configured to collect the sample from the aerosol cloud 102 can be operatively connected to one or both of the vehicle 104 and the detection module 108 and can extend generally outwardly therefrom. In embodiments, e.g., as shown in the enlarged Fig. 2, the collection arm 110 can form shroud 112 defining a plenum 114 between one or more outward projections 112a, 112b of the collection arm 110 and the detection module 108. The shroud 112 can include an opening 116 configured to receive aerosol particles from ambient A, including aerosol particles from the aerosol cloud 102. The collection arm 110 can also include a collection tube 118 fluidly connected to the plenum 114 to collect the sample from the plenum 114. In embodiments, the length I of the collection tube and collection arm 110 as a whole, can be configured to provide a sufficient standoff distance D between the vehicle/detection module 104, 108 and the aerosol cloud 102 such that exposure of the vehicle 104 and detection module 108 to the aerosol cloud 102 is minimized. The length I of the collection arm 110 can be the minimum sufficient standoff distance D to minimize exposure, however, the standoff distance may change as the aerosol cloud 102 moves or disperses and as the vehicle/detection module 104, 108 performs different tasks. In embodiments, the standoff distance D can be determined as a function of the mobile platform 104 and its components. For example, if the mobile platform 104 includes a rotor craft (e.g., a drone), the standoff distance D can be about 10 feet, or at least far enough to avoid rotor or prop wash and drift of contamination to the optical detection module 108. For a land craft, or watercraft, the standoff distance D may be greater or less than 40 feet.

With reference to Figs. 1 and 2, in embodiments, a shape of the collection arm 110 is configured to enclose (e.g., fully enclose) a path 120 of optical signals issued from and received by the detection module 108 within the plenum 114, as discussed further below. In embodiments (e.g., as shown), the collection tube 118 can be L-shaped, having a long side 122 and a short end 124. As used herein, long and short are with reference to each other, for example the long side 122 is longer than the short end 124, and the short end 124 is shorter than the long side 122. The long side 122 can form an upper boundary U of the plenum 114 and the short end can form an outer boundary O of the plenum 114. The outward projection 112a of the detection module 108 can form a lower boundary L of the plenum 114 while the detection module 108 itself forms the final boundary of the plenum 114. In certain embodiments, the short end 124 of the collection tube 118 can be downward facing relative to the direction of gravity G (e.g., when the vehicle 104 is upright) and an inlet 126 of the collection tube 118 can be disposed at or near the short end 124. In embodiments, the inlet 126 of the collection tube 118 can be disposed on an inner surface 128 of the shroud 112 (e.g., facing into the plenum 114 and facing the detection module 108), proximate the opening 116 in the shroud 112, so that the collection tube 118 is fluidly connected to the plenum 114 and to ambient A through the opening 116 in the plenum 114. In embodiments, the inlet 126 of the collection tube 118 can be on an opposite end of the long side 122 of the collection tube 118 than the detection module 108.

A collection medium 130 can be disposed at the inlet 126 of the collection tube 118 configured to collect aerosol particles 132 from the aerosol cloud 102 that have entered the plenum 114 and condense the aerosol particles on or in the collection medium 130. In embodiments, the collection medium 130 can include a filter medium such as a porous medium, including filter paper for example.

With continued reference to Fig. 2, in certain embodiments, collection of the sample can be passive, wherein the aerosol particles 132 naturally enter the plenum 114 and the collection tube 118 onto the collection medium 130 simply by virtue of the collection arm 110 entering the aerosol cloud 102. In certain embodiments, a pump 134 can be disposed in the collection tube 118 downstream of the collection medium 130 configured to pull or draw aerosol particles through the opening 116 in the plenum 114, through the inlet 126 of the collection tube 118, and into or onto the collection medium 130. In embodiments, the vehicle 104 and detection module 108 can be coupled so as to define a fluid channel 136 therebetween to allow particles from the plenum 134 to actively or passively exhaust or otherwise vent through an outlet 138 of the collection tube 118 and back to ambient A. In embodiments, collection medium 130 can fill the entirety of the inlet 126 of the collection tube 118 so that when fluid is exhausted or vented through the fluid channel 136, neither of the vehicle 104 or detection module 108 are exposed to the aerosol particles from the aerosol cloud 102.

Referring still to Fig, 2, in embodiments, the detection module 108 can include an optical detection module. The optical detection module 108 can include a radiation source 140 (e.g., deep UV, infrared, quantum cascade lasers, or the like) configured to transmit an interrogation beam 142 through the plenum 114 to interrogate the collection medium 130. The optical detection module 108 can also include a radiation receiver 144 configured to receive a return signal 146 through the plenum 114 from the collection medium 130. The interrogation beam 142 will interrogate the sample and return one or more wavelengths (the same or different than the interrogation beam), the wavelengths being a function of the chemical composition of the sample (e.g., as in Raman spectroscopy). In embodiments, the optical detection module 108 can also include a composition determination module 148 configured to receive data from the radiation receiver 144 (e.g., an amount of light and wavelength) to determine the chemical composition of the particles in the collection medium 130, and thus the chemical composition of the aerosol cloud 102. In certain embodiments, the determination module 148 can include a spectrometer (e.g., a Raman spectrometer).

Because the composition of the aerosol cloud 102 is unknown at the time of launching the vehicle 104 and before the first sample is interrogated, the standoff distance D between the UAV 104 and the cloud should be sufficient such that exposure of the UAV 104 to the aerosol cloud 102 is minimized in the event the aerosol cloud 102 does contain a contaminant or hazardous material. Once the chemical composition of the aerosol cloud 102 has been determined (even if only preliminarily), if the aerosol cloud 102 does contain a contaminant, the standoff distance D must be maintained while sampling continues and of the cloud 102 begins to move or disperse.

Referring now to Figs. 1-4, in embodiments, the vehicle 104 can further include a communications module 150 configured to communicate with a controller 152 to guide the vehicle 104 to one or more respective positions in space relative to the aerosol cloud 102 (e.g., geographical coordinates of interest, for example the suspected location of the aerosol cloud 102). For example, at launch the controller 152 can communicate with the communications module 150 to place the vehicle 104 at or near (e.g., at an edge 102' of) the aerosol cloud 102. In embodiments, the communications module 150 can also be configured to communicate the composition of the aerosol cloud 102 to the controller 152 and/or a user 155, for example to notify remote personnel of potential hazards with respect to the composition aerosol cloud 102. In certain embodiments, the controller 136 can be or include a remote and/or on-ground controller and in certain embodiments may be included on or in a base unit 154.

In embodiments, the vehicle 104 can also include a respective navigation module 156 configured to guide the vehicle 104 to one or more positions in space relative to the aerosol cloud 102 without communication from remote or on ground controller (e.g. controller 152). For example, in certain embodiments, the vehicle 104 can include at least some amount autonomous control, such as with respect to positioning the vehicle 104 relative to the aerosol cloud 102 (e.g., based on sensor data from a sensor in the collection arm or detection module) and/or positioning the vehicle 104 relative to other nearby vehicles included in the system 100, if any. This can include communication between the vehicle 104 and the controller 152 to position the vehicle 104 at a given location to maintain the standoff distance D as the aerosol cloud moves or disperses, and/or communications between other nearby vehicles to avoid collisions, among others.

Any suitable or appropriate communication system between the vehicle 104 and and a grounded or otherwise remote controller 152 is contemplated herein. For example, the vehicle 104 can have an onboard sensing or positioning system capable of locating nearby vehicles and maintaining a given distance. It is also contemplated that the vehicle 104 can include satellite guidance.

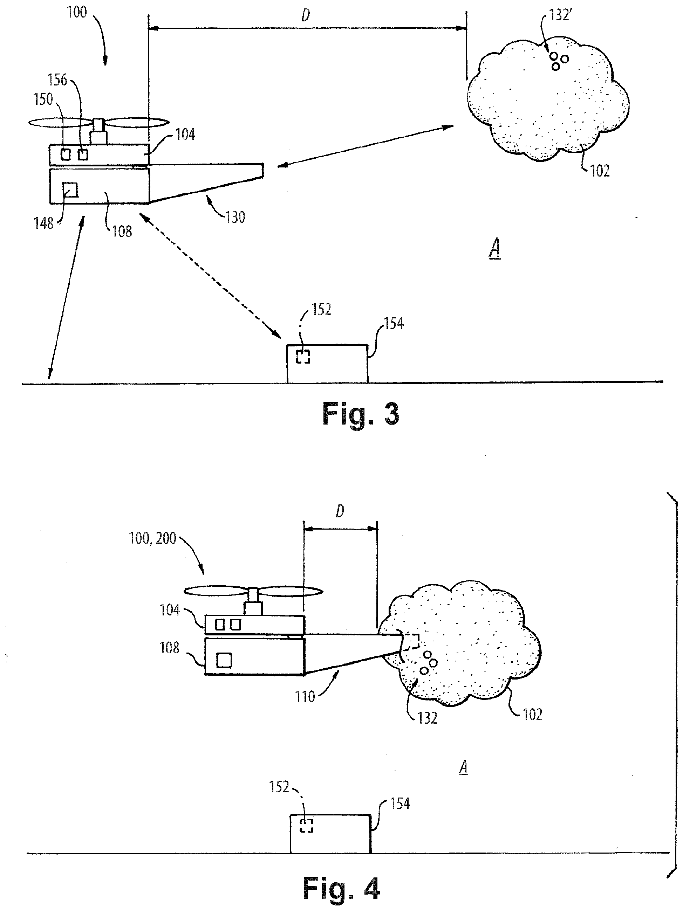

In accordance with at least one aspect of this disclosure, e.g., as shown in Figs. 3 and 4, a method 200 can include remotely collecting a sample material from an aerosol cloud (e.g., cloud 102) in a collection medium (e.g., medium 130) and condensing the aerosol particles on or in the collection medium. The method can include optically interrogating the particles in the collection medium, determining a chemical composition of the particles in the collection medium, and communicating the chemical composition to a user (e.g., user 155) and/or a controller (e.g., 152).

In embodiments, remotely collecting the sample can further include detecting an edge of the aerosol cloud (e.g., edge 102'), positioning a vehicle having an optical detection module and a collection arm operatively connected thereto (e.g., vehicle 104 having detection module 108 and collection arm 110) proximate the aerosol cloud separated by a standoff distance such that only a portion of the collection arm enters the aerosol cloud and no portion of the vehicle or optical detection module enters the aerosol cloud. In embodiments, collecting the sample can also include drawing (e.g., with a pump 134) a portion of the aerosol cloud through an inlet of a collection tube (e.g., collection tube 118) of the collection arm. In embodiments, the method can include passively collecting the sample.

In embodiments, the method can also include moving the vehicle away from the aerosol cloud while the optical detection system determines the chemical composition, re-positioning the vehicle proximate the aerosol cloud and repeating the method (e.g., if more sampling is needed) or homing the vehicle to a grounded or local base (e.g., base 154). Once on homed, the method can include initiating a decontamination protocol based at least in part of the chemical composition of the areosol particles to decontaminate or destroy any vehicles or parts that are too contaminated for reuse or for archiving.

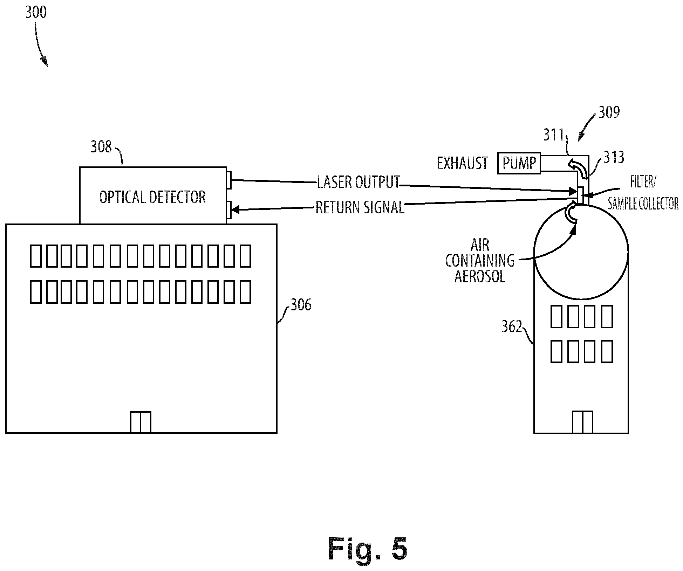

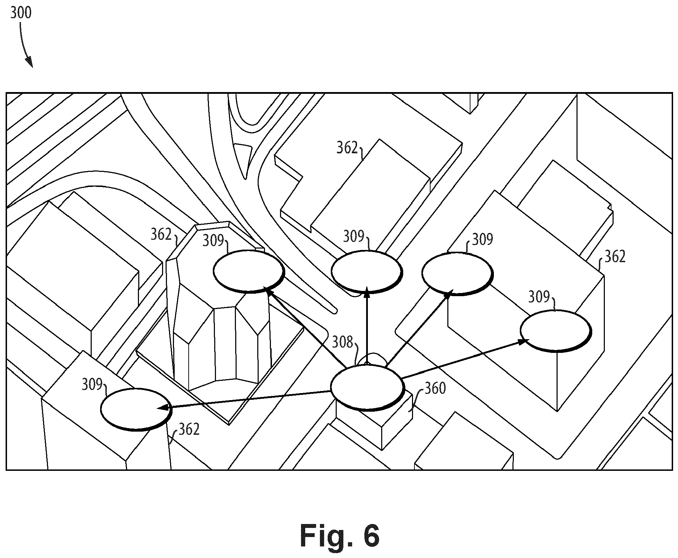

In certain embodiments, as shown in Figs. 5 and 6, a system 300 can be a stationary aerosol collection and detection system 300. The system 300 can include an optical detection module 308 and a collection system 309, physically separate from one another. The optical detection system 308 can be the same or similar to the optical detection system 108 as described above. The collection system 309 can include a collection arm 310 and collection tube 318 the same or similar to the collection arm 110 and collection tube 118 as described above. The system 300 can operate in the same or similar manner as to that described above with respect to system 100.

In certain embodiments, e.g., as shown, the optical detection system 308 can be included on or in a first stationary structure 360, and the collection system 309 can be included on or in a second stationary structure 362, physically separate from the first stationary structure 360 to collect aerosol samples surrounding the structures 360, 362 or from within the structures 306, 362 (e.g., in ductwork). The structure can include a building, for example if in an urban environment (e.g., as shown in Fig. 6), or any other suitable structure/fixture for a given environment. In certain embodiments, the optical detection module 308 can be included on or in the first stationary structure 360 while the collection system 309 can be included on a mobile platform, such as vehicle 304. In certain embodiments, the opposite can be true, where the optical detection module 308 can be included on a mobile platform such as vehicle 304, while the collection system 309 can be included on or in the second stationary structure 362. In embodiments, the system 300 can include any suitable number of optical detection modules 308 and any suitable number of collection system 309. In certain embodiments, the system 300 can include more collection system 309 than optical detection modules 308, and in such embodiments, the optical detection modules 308 can be centrally located (e.g., surrounded by) a plurality of collection systems 309. In certain embodiments, the first and second structures 360, 362 can include any suitable number of optical detection modules 308 or collection systems 309. For example the first structure 362 can include an optical detection module 308 and multiple collection systems 309. In certain embodiments, the second structure can include multiple collection systems 309 and no optical detection modules 308. Any suitable arrangement of optical detection modules 308 and collection systems 309 is contemplated herein.

Embodiments can include a mobile or stationary (or combination of the two) aerosol detection system, having a collection payload platform and an optically based detection system. An arm or other extension to collect aerosolized material onto a surface which can then be interrogated by the system can be included on or attached to the vehicle in mobile embodiments. The arm can include an active air flow to draw aerosol-containing air onto a front surface of the filter surface where some of the aerosol is trapped. Aerosol may accumulate for a period of time to enhance detection limits if needed, or the system can detect in real time. In certain embodiments, the detection system as shown and described can convert a liquid aerosol detection into a hard surface detection, for example by collecting the aerosol onto a hard surface (e.g., filter paper or the like). In embodiments, utilizing a long collection arm can minimize exposure of the detection system and the vehicle to the contaminating aerosol.

Any suitable or appropriate communication system between the vehicle and other nearby vehicles, or the vehicle and a grounded or otherwise remote controller is contemplated herein. For example, each vehicle can have an onboard sensing or positioning system to be able to locate nearby vehicles. Embodiments can include an aerosol detection systems using certain vehicles, such as unmanned aerial vehicles ("UAVs"), for example chemical and/or biological aerosol detection systems.

As will be appreciated by those skilled in the art, aspects of the present disclosure may be embodied as a system, method or computer program product. Accordingly, aspects of this disclosure may take the form of an entirely hardware embodiment, an entirely software embodiment (including firmware, resident software, micro-code, etc.), or an embodiment combining software and hardware aspects, all possibilities of which can be referred to herein as a "circuit," "module," or "system." A "circuit," "module," or "system" can include one or more portions of one or more separate physical hardware and/or software components that can together perform the disclosed function of the "circuit," "module," or "system", or a "circuit," "module," or "system" can be a single self-contained unit (e.g., of hardware and/or software). Furthermore, aspects of this disclosure may take the form of a computer program product embodied in one or more computer readable medium(s) having computer readable program code embodied thereon.

Any combination of one or more computer readable medium(s) may be utilized. The computer readable medium may be a computer readable signal medium or a computer readable storage medium. A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Embodiments may include or utilize computer program instructions. These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Those having ordinary skill in the art understand that any numerical values disclosed herein can be exact values or can be values within a range. Further, any terms of approximation (e.g., "about", "approximately", "around") used in this disclosure can mean the stated value within a range. For example, in certain embodiments, the range can be within (plus or minus) 20%, or within 10%, or within 5%, or within 2%, or within any other suitable percentage or number as appreciated by those having ordinary skill in the art (e.g., for known tolerance limits or error ranges).

The articles "a", "an", and "the" as used herein and in the appended claims are used herein to refer to one or to more than one (i.e., to at least one) of the grammatical object of the article unless the context clearly indicates otherwise. By way of example, "an element" means one element or more than one element.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of."

Any suitable combination(s) of any disclosed embodiments and/or any suitable portion(s) thereof are contemplated herein as appreciated by those having ordinary skill in the art in view of this disclosure.

The embodiments of the present disclosure, as described above and shown in the drawings, provide for improvement in the art to which they pertain. While the apparatus and methods of the subject disclosure have been shown and described, those skilled in the art will readily appreciate that changes and/or modifications may be made thereto without departing from the scope of the subject disclosure.

## Claims

1. A system, comprising:
a collection arm (110) configured to collect an aerosol sample from a cloud; and
a detection module (108) operatively connected to the collection arm at an opposite end of the collection arm than the sample, configured to analyze a composition of the sample from the cloud.

2. The system of claim 1, wherein one or more of the collection arm (110) and/or the detection module (108) is configured to couple to a mobile platform (104).

3. The system of claim 1 or 2, wherein the collection arm (110) defines:
a plenum (114) between an outward projection (112a, 112b) of the collection arm and the detection module (108), the collection arm having an opening (116) configured to receive aerosol particles from the cloud and into the plenum; and
a collection tube (118) fluidly connected to the plenum to collect the sample.

4. The system of claim 3, further comprising a collection medium (130) disposed at an inlet (126) of the collection tube (118) configured to collect aerosol particles from the cloud and condense the aerosol particles onto the collection medium; and optionally further comprising a pump (134) disposed in the collection tube (118) downstream of the collection medium (130) configured to draw the aerosol particles through the opening in the collection arm, through the inlet (126) of the collection tube, and into or onto the collection medium.

5. The system of any preceding claim, wherein the detection module (108) includes an optical detection module (308); and optionally, wherein the optical detection module includes a radiation source (140) configured to transmit an interrogation beam (142) through the plenum (114) to interrogate the collection medium (130).

6. The system of claim 5, wherein the optical detection module (308) further includes a radiation receiver (144) configured to receive a return signal through the plenum (114) from the collection medium (130); and optionally wherein the optical detection module further includes a composition determination module (148) configured to receive data from the radiation receiver (144) to determine a chemical composition of the aerosol particles in the collection medium (130).

7. The system of any of claims 3 to 6, wherein a length of the collection tube (118) is configured to provide sufficient standoff distance between the detection module (108) and the cloud such that exposure of the detection module to the cloud is minimized.

8. The system of claim 6 or 7, wherein a shape of the collection arm (110) is configured to enclose a path of the interrogation beam (142) and a path of the return signal within the plenum (114).

9. The system of any of claims 4 to 8, wherein the collection tube (118) is L-shaped having a short end and a long side wherein the inlet (126) of the collection tube is disposed in the short end of the collection tube and on an opposite end of the long side of the collection tube than the detection module (108); and optionally wherein the inlet (126) of the collection tube is disposed on an inner surface of the collection arm, proximate the opening (116) in the collection arm, wherein the collection medium is disposed in the inlet of the collection tube.

10. The system of any of claims 2 to 9, wherein the mobile platform (104) further includes a communications module (150) configured to communicate with a controller (152) to guide the mobile platform to one or more positions in space relative to the cloud.

11. The system of claim 10, wherein the controller (152) includes a remote and/or on-ground controller; and/or wherein the communications module (150) is operatively connected to the optical detection module (308) and further configured to communicate the composition of the cloud to the controller (152) and/or a user.

12. The system of any of claims 2 to 11, wherein the mobile platform (104) further includes a navigation module (156) configured to guide the mobile platform to one or more positions in space relative to the cloud without communication from a remote or on-ground controller.

13. A method, comprising:
remotely collecting an aerosolized sample material from an cloud in a collection medium;
optically interrogating the sample material in the collection medium;
determining a chemical composition of the sample material in the collection medium; and
communicating the chemical composition to a user.

14. The method of claim 13, wherein remotely collecting further includes:
detecting an edge of the aerosol cloud;
positioning a mobile platform having an optical detection module and a collection arm operatively connected thereto proximate the aerosol cloud separated by a standoff distance such that only a portion of the collection arm enters the aerosol cloud and no portion of the mobile platform or optical detection module enters the aerosol cloud; and
drawing a portion of the aerosol cloud through an inlet of a collection tube of the collection arm, wherein the collection medium is disposed at an inlet of the collection tube.

15. The method of claim 14, further comprising:
re-positioning the mobile platform proximate the aerosol cloud and repeating the method or homing the mobile platform to a base; and
initiating a decontamination protocol based at least in part of the chemical composition of the sample material.
